Europäisches Patentamt

(19) European Patent Office

Office européen des brevets

(11) Numéro de publication : **0 459 861 A1**

(12) # DEMANDE DE BREVET EUROPEEN

(21) Numéro de dépôt : 91401293.5

(22) Date de dépôt : 17.05.91

(51) Int. Cl.[5] : **C07B 41/06, C07B 41/02, C07C 49/385, C07C 49/78, C07C 47/54, C07C 35/00, C07C 33/22, C07C 45/28, C07C 29/48**

(30) Priorité : 31.05.90 FR 9007046

(43) Date de publication de la demande :
04.12.91 Bulletin 91/49

(84) Etats contractants désignés :
AT BE CH DE DK ES FR GB GR IT LI LU NL SE

(71) Demandeur : ATOCHEM
4 & 8, Cours Michelet La Défense 10
F-92800 Puteaux (FR)

(72) Inventeur : Larpent, Chantal, DE CHIMIE DE
RENNES
Avenue du Général Leclerc
F-35700 Rennes-Beaulieu (FR)
Inventeur : Patin, Henri, Prof., DE CHIMIE DE
RENNES
Avenue du Général Leclerc
F-35700 Rennes-Beaulieu (FR)

(74) Mandataire : Rochet, Michel et al
ATOCHEM Département Propriété Industrielle
F-92091 Paris la Défense Cédex 42 (FR)

(54) ACTIVATION OXYDANTE DE LIAISONS CARBONE-HYDROGèNE PAR LE PEROXYDE D'HYDROGèNE, MILIEU ET PROCéDé POUR LA RéALISER.

(57)     Activation oxydante de liaisons carbone-hydrogène dont l'atome de carbone est à l'état d'hybridation de type $sp^3$ dans un hydrocarbure par action de $H_2O_2$ sur l'hydrocarbure dans le milieu résultant, à l'état de microémulsion, du mélange de deux microémulsions eau dans hydrocarbure, l'une contenant du peroxyde d'hydrogène, l'autre un sel ou un dérivé oxygéné colloïdal d'un métal de degré d'oxydation multiple et capable de conduire à des oxydes hydratés.

EP 0 459 861 A1

La présente invention concerne l'activation oxydante de liaisons C-H dont l'atome de carbone est à l'état d'hybridation de type sp³ dans un hydrocarbure.

Dans l'oxydation de substrats organiques, le peroxyde d'hydrogène peut agir en l'absence d'un catalyseur, par exemple quand il agit en combinaison avec l'hypochlorite de sodium ou le brome en milieu alcalin, ou en présence d'un catalyseur comme un sel ou un oxyde métallique ou métalloïdique.

C'est ainsi qu'ont pu être oxydés des alcènes et des composés aromatiques.

Par exemple, l'oxydation de composés de ces deux catégories par le peroxyde d'hydrogène en milieu alcalin en présence d'un molybdate ou d'un tungstate de métal alcalin comme catalyseur, est décrite dans la demande de brevet européen n°0288337 : le peroxyde d'hydrogène en solution aqueuse est ajouté au composé à oxyder dissous dans une microémulsion formée au préalable par addition d'une solution aqueuse du catalyseur dans une suspension contenant un solvant, un tensioactif insoluble dans le solvant et éventuellement un cotensioactif. Un alcane ou un composé aromatique comme le toluène conviennent comme solvant récupéré en fin d'opération.

L'oxydation d'alcanes ou de cyclanes à l'aide de peroxyde d'hydrogène est restée quant à elle très liée jusqu'à ce jour à l'emploi de milieux très fortement acides. Le résultat en est le plus généralement une coupure de la chaîne hydrocarbonée, la formation de mélanges de produits isomères et de sous-produits indésirables.

Par exemple le cyclohexane est dégradé en quasi-totalité par le peroxyde d'hydrogène en présence du couple HF, BF₃ dès la température ambiante selon R. WALDEN et Coll., J. Chem. Soc., 4707 (1964).

L'oxydation d'alcanes à l'aide de peroxyde d'hydrogène a été aussi réalisée grâce à des systèmes tels que ceux dits de FENTON, d'UDENFRIEND, de GIF, respectivement décrits dans J. Chem. Soc., 65, 899 (1884), J. Biol. Chem. 208, 731 (1954), Tetrahedron Lett. 31, n°12, 1657-1660 (1990). Pour que ces systèmes puissent fonctionner, il doit être fait appel à un tiers corps, comme par exemple l'acétonitrile, la pyridine ou ses dérivés, capable de former avec l'eau un solvant mixte à la fois des constituants du système, qui sont solubles dans l'eau, et de l'hydrocarbure à oxyder, qui lui ne l'est pas. De plus certaines espèces réactives sont peu stables et peuvent être immédiatement consommées dans d'autres réactions que celle désirée, par exemple avec le solvant.

Il a maintenant été trouvé un procédé d'oxydation d'alcanes ou de cyclanes, et même plus largement un procédé d'activation oxydante de liaisons C-H dont l'atome de carbone est à l'état d'hybridation de type sp³ dans un hydrocarbure, par action de peroxyde d'hydrogène sur l'hydrocarbure en présence d'un catalyseur, qui ne fait pas appel aux superacides, permet de se dispenser d'un solvant de même que d'un cotensioactif, et se traduit par une sélectivité de l'activation de la liaison C-H en alcool et/ou cétone.

Un tel procédé constitue un premier objet de la présente invention.

Un hydrocarbure dont au moins un atome de carbone porteur de liaisons C-H est dans l'état d'hybridation de type sp³ est désigné dans tout ce qui suit du seul terme d'hydrocarbure. La liaison C-H activée selon le procédé de l'invention peut faire partie d'une chaîne hydrocarbonée acyclique ou cyclique et par exemple être située dans un alcane ou un cyclane.

Le procédé de la présente invention est caractérisé en ce que le peroxyde d'hydrogène agit sur l'hydrocarbure dans le milieu résultant à l'état de microémulsion du mélange de deux microémulsions de type "eau dans huile" avec comme huile l'hydrocarbure, la première contenant du peroxyde d'hydrogène, la seconde un sel ou un dérivé oxygéné colloïdal d'un métal de degré d'oxydation multiple et capable de conduire à des oxydes hydratés.

Dans la définition ci-dessus du procédé de l'invention comme dans tout ce qui suit, on entend par microémulsion d'eau dans l'hydrocarbure un mélange qui se présente comme une solution monophasique, limpide, stable thermodynamiquement et optiquement isotrope, et dont les constituants de base sont l'eau, l'hydrocarbure, composés insolubles l'un dans l'autre, et un agent tensio-actif.

Les microémulsions dont il est question dans le procédé selon l'invention sont dites du type "eau dans huile" parce que la phase continue est l'huile, c'est-à-dire ici l'hydrocarbure. Elles sont désignées dans tout ce qui suit par le seul terme de microémulsion.

La microémulsion contenant le peroxyde d'hydrogène mais pas le composé du métal, est désignée dans tout ce qui suit par (E/H)₁.

La microémulsion contenant le composé du métal mais pas le peroxyde d'hydrogène est désignée dans tout ce qui suit par (E/H)₂.

Parmi les hydrocarbures auxquels s'applique le procédé de l'invention, on peut citer à titre d'exemple, l'octane, le cyclooctane, le décahydronaphtalène ou décaline, l'éthylbenzène, l'isopropylbenzène ou cumène, le tétrahydro-1,2,3,4 napthalène ou tétraline.

Les hydrocarbures auxquels s'appliquent le plus aisément le procédé de l'invention sont ceux qui sont liquides, soit en l'état, soit, le cas échéant, par solubilisation dans un solvant, à une température comprise entre environ 10°C et la température d'ébullition du plus volatil des constituants du milieu dans lequel le peroxyde d'hydrogène agit sur l'hydrocarbure, c'est-à-dire une fois réalisé le mélange de (E/H)₁ avec (E/H)₂. Cet intervalle de température, dans lequel est choisie la température à laquelle le peroxyde d'hydrogène agit sur l'hydrocarbure, est le plus généralement d'environ 5°C à 80°C. Le choix de la température dépend essentiellement de la nature de l'hydrocarbure et éventuellement de celle du solvant qui l'accompagne que ce soit nécessairement ou non. Les hydrocarbures auxquels il est fait ainsi référence peuvent comprendre de 4 à 30 atomes de carbone dans leur molécule.

L'agent tensio-actif peut être choisi parmi les agents tensio-actifs ioniques usuels notamment les tensio-actifs anioniques usuels tels que les alkylsulfates et alkylsulfonates, les alkylarylsulfates et alkylarylsulfonates, les sels alcalins d'acides organiques sulfatés ou sulfonés comme par exemple le dioctylsulfosuccinate de sodium, ainsi que les tensioactifs cationiques usuels tels que les sels d'ammonium quaternaire, comme par exemple un halogénure de dodécyltriméthylammonium.

Pour ce qui concerne les microémulsions on se reportera par exemple à BERTHOD Alain, Journal de Chimie Physique, 80, n°5, 407-424 (1983), ou à BOUTONNET Magali et col., Colloids and Surfaces, 5, 209-225 (1982).

Comme exemples de métaux de degré d'oxydation multiple et capables de conduire à des oxydes hydratés on peut citer le fer, le cuivre, le ruthénium, le rhodium, le molybdène, le titane.

Les sels de tels métaux qui sont utilisés dans le procédé de l'invention sont des sels solubles en milieu aqueux, d'acides minéraux ou organiques, par exemple un chlorure, un sulfate, un nitrate, un sel double de métal à un de ses degrés d'oxydation possibles. Dans le cas du fer ce sera par exemple le chlorure ferrique $FeCl_3$, le nitrate ferrique ou bien le sulfate ferreux par exemple sous forme de sel de Mohr $FeSO_4$ $(NH_4)_2$ $SO_4$, $6H_2O$.

Par dérivé oxygéné colloïdal du métal dans la deuxième microémulsion on entend un oxyde, ou un hydroxyde, ou un oxyhydroxyde dudit métal ou un mélange de ces formes. Ce dérivé oxygéné colloïdal est obtenu d'abord sous forme de dispersion colloïdale aqueuse ou hydrodispersion colloïdale selon une technique connue comme par exemple une technique extraite des articles de E. MATIJEVIC et coll., J. Coll. Int. Sc., 109, 57 (1986) ou Colloid & Polymer Sci., 265, 155 (1987) soit sous forme de dispersion colloïdale ou microémulsion par hydrolyse du sel métallique par une base inorganique, tel que décrit par S. Mann et coll., I. Coll. Interf. Science, 122, 326, 1988 pour ce qui concerne un dérivé oxygéné colloïdal de fer. Par exemple un dérivé oxygéné colloïdal du fer est obtenu par vieillissememement avec chauffage par exemple à une température de l'ordre de 75°C à 100°C, d'une solution aqueuse de $FeCl_3$ acidifiée par HCl et dans laquelle l'hydrolyse du chlorure ferrique est initialement évitée à température ambiante du fait de cette acidification.

Le solvant organique qui accompagne éventuellement l'hydrocarbure ne doit évidemment pas rompre l'état de microémulsion ni interférer sur l'action du peroxyde d'hydrogène. Ce peut être un hydrocarbure aromatique, le benzène le plus généralement, ou un hydrocarbure halogéné tel que le dichlorométhane ou éthane.

Le peroxyde d'hydrogène est mis en oeuvre sous forme de solution aqueuse, par exemple une solution aqueuse contenant 30 %, 50 % ou 70 % de peroxyde d'hydrogène en poids. Il est préférable d'utiliser le peroxyde d'hydrogène sous la forme la plus concentrée possible compatible avec la sécurité pour avoir un meilleur contrôle de la stabilité de l'état de microémulsion du milieu résultant du mélange de (E/H)₁ avec (E/H)₂.

Le pH dudit milieu est de préférence inférieur ou égal à 7. Une valeur de ce pH compris entre 2 et 5 convient généralement bien. Par exemple, lorsqu'est choisi un sel de fer comme sel de métal, un pH égal à 3 ou peu éloigné de 3 permet de concilier vitesse de réaction et efficacité du peroxyde d'hydrogène. Une telle valeur de pH est commodément obtenue dans ce cas puisqu'elle résulte naturellement du mélange de (E/H)₁ avec (E/H)₂.

L'action du peroxyde d'hydrogène sur l'hydrocarbure s'exerce de préférence sous atmosphère inerte, par exemple sous azote, plutôt que sous atmosphère oxygénée comme l'air, pour orienter l'oxydation de l'hydrocarbure vers la formation d'une cétone plutôt que vers celle d'un mélange d'alcool et de cétone.

L'addition de peroxyde d'hydrogène en solution aqueuse au milieu résultant du mélange de (E/H)₁ avec (E/H)₂ permet de poursuivre la transformation de l'hydrocarbure en alcool et/ou cétone au-delà de celle assurée du fait du seul peroxyde d'hydrogène apporté par (E/H)₁, et indépendamment alors de la persistance ou non de l'état de microémulsion initial dudit milieu.

Un mode de préparation de (E/H)₁, le plus généralement choisi, consiste à mélanger, à une température comprise par exemple entre 5°C et 60°C, une solution de l'agent tensioactif dans l'hydrocarbure avec une solution aqueuse de peroxyde d'hydrogène. Le mélange est réalisé de préférence par addition de la seconde solution dans la première.

Un mode opératoire de (E/H)₂, le plus généralement choisi consiste à mélanger, à une température comprise par exemple entre 5°C et 80°C, une solution de l'agent tensio-actif dans l'hydrocarbure soit avec une solution aqueuse de sel de métal suivie de l'addition éventuelle d'une base minérale, soit avec un dérivé oxygéné colloïdal du métal à l'état de dispersion colloïdale aqueuse. Le mélange est réalisé de préférence par addition de la solution de sel ou bien de la dispersion colloïdale dans la solution de l'agent tensio-actif dans l'hydrocarbure.

Les proportions des constituants, compatibles avec la formation de chacune des microémulsions (E/H)$_1$ et (E/H)$_2$, découlent évidemment de l'établissement cas par cas des diagrammes de phases respectifs.

Les proportions relatives de (E/H)$_1$ et de (E/H)$_2$ pour leur mélange doivent être compatibles avec l'obtention d'une microémulsion comme résultat dudit mélange. Elles découlent de la connaissance de l'incidence de la variation des proportions des différents constituants sur ce résultat.

Dans un tel cadre pourront être déterminés les choix convenant le mieux à l'oxydation d'un hydrocarbure particulier.

A titre indicatif, quand le sel de métal dans (E/H)$_2$ est un sel de fer et l'agent tensio-actif le dioctylsulfosuccinate de sodium, la solution de l'agent tensio-actif dans l'hydrocarbure peut contenir en poids une quantité comprise entre environ 5 % et la limite de solubilité dudit agent dans l'hydrocarbure, qu'il s'agisse de préparer (E/H)$_1$ ou (E/H)$_2$.

Pour la préparation de (E/H)$_2$ la solution aqueuse de sel et de fer peut contenir en poids jusqu'à 10 % de ce sel et ladite solution être ajoutée à la solution d'agent tensioactif dans l'hydrocarbure à raison de 0,01 % à 20 % en poids par rapport à celle-ci. Il en est de même dans le cas où la solution de sel est remplacée par la dispersion colloïdale du dérivé oxygéné du métal. La quantité d'eau dans (E/H)$_1$ est telle que, par mélange volume à volume de (E/H)$_2$ avec (E/H)$_2$, elle représente jusqu'à environ 2 fois celle apportée par (E/H)$_2$.

Le caractère catalytique de l'oxydation de l'hydrocarbure selon l'invention a été vérifié.

Il a par ailleurs été constaté que le peroxyde d'hydrogène:

. n'agit pas sur l'hydrocarbure au sein de (E/H)$_1$,

. n'agit pas de façon sensible sur l'hydrocarbure s'il est ajouté sous forme de solution aqueuse à (E/H)$_2$,

. n'agit pas de façon sensible sur l'hydrocarbure si le sel de métal est sous forme de solution aqueuse quand il est ajouté à (E/H)$_1$,

. n'agit sur l'hydrocarbure qu'à partir du moment où (E/H)$_1$ et (E/H)$_2$ ont été mélangées,

. agit sur l'hydrocarbure s'il est mélangé sous forme de solution aqueuse au mélange de (E/H)$_1$ avec (E/H)$_2$ même après une durée longue sans addition de peroxyde d'hydrogène au dit mélange, plusieurs jours par exemple. C'est le signe de l'existence dans le milieu résultant à l'état de microémulsion du mélange de (E/H)$_1$ avec (E/H)$_2$ d'une entité catalytique particulièrement stable.

Le mode de formation du milieu dans lequel se révèlent l'activité et la stabilité de cette entité catalytique constitue le deuxième objet de la présente invention. Ce mode de formation avec ses caractéristiques ont été rapportés plus avant dans ce texte. Un troisième objet de l'invention est la microémulsion résultant du mélange de (E/H)$_1$ avec (E/H)$_2$.

Sans que cela puisse en rien limiter la présente invention le fait que l'entité catalytique dont il est fait mention ici est partie intégrante d'une microémulsion indique qu'elle contient le métal vraisemblablement d'un dérivé à l'état colloïdal.

Les exemples suivants, donnés à titre indicatif mais non limitatif illustrent l'invention.

Exemple 1 :

L'hydrocarbure soumis à l'action du peroxyde d'hydrogène est le cyclooctane.

1,2 g. de dioctylsulfosuccinate de sodium sont dissous dans 5 cm$^3$ de cyclooctane par agitation à température ambiante.

A cette solution est ajouté à température ambiante 1 cm$^3$ de solution aqueuse de peroxyde d'hydrogène H$_2$O$_2$ à 30 % en poids de H$_2$O$_2$ (9,8 millimoles de H$_2$O$_2$), pour obtenir sur simple mélange la microémulsion (E/H)$_1$.

A un même volume de la même solution de dioctylsulfosuccinate de sodium que ci-dessus pour préparer (E/H)$_1$, on ajoute goutte à goutte et sous agitation 0,5 cm$^3$ d'une solution aqueuse à 5 % en poids de sel de Mohr FeSO$_4$, (NH$_4$)$_2$SO$_4$, 6H$_2$O, pour obtenir la microémulsion (E/H)$_2$.

(E/H)$_1$ et (E/H)$_2$ sont sous atmosphère d'azote.

(E/H)$_1$ et (E/H)$_2$ sont mélangées en étant maintenues sous atmosphère d'azote de même que le milieu résultant de leur mélange.

L'évolution dudit mélange dans le temps à 25°C est suivie par chromatographie en phase gazeuse.

Après une durée de 24 heures à partir du moment où (E/H)$_1$ et (E/H)$_2$ sont mélangées, il s'est formé 0,45 millimole de cyclooctanone, ce qui correspond à une conversion du cyclooctane engagé (74,5 millimoles) égale à 0,6 %. La cyclooctanone est le seul composé décelable formé à partir du cyclooctane.

Après que les 24 heures ci-dessus aient été atteintes, et toutes les 3 heures à partir de ce moment, on ajoute au milieu de réaction 0,25 cm$^3$ de solution aqueuse de H$_2$O$_2$ à 30 % en poids de H$_2$O$_2$. Lorsqu'ainsi 8 cm$^3$ de ladite solution ont été ajoutés (78,4 millimoles de H$_2$O$_2$), il s'est formé une quantité supplémentaire de cyclooctanone égale à 10,75 millimoles.

11,2 millimoles de cyclooctanone se sont donc formées à partir du moment où $(E/H)_1$ et $(E/H)_2$ ont été mélangées, ce qui correspond à une conversion sélective du cyclooctane en cyclooctanone égale à 15 %.

Les produits (cyclooctane non réagi et cyclooctanone formée) sont séparés du milieu réactionnel par fixation du tensioactif ionique sur une résine échangeuse d'ions en opérant comme suit : le mélange réactionnel est dilué dans 50 ml d'eau et 50 ml de méthanol et la solution résultante est ensuite percolée à travers une colonne contenant 50 g d'une résine échangeuse d'anions (Amberlite IR 45). L'étude par chromatrographie couche mince montre que le tensioactif a été retenu en totalité. Les produits sont ensuite extraits du filtrat par un solvant organique. Le dioctylsulfosuccinate retenu sur la résine est ensuite récupéré par lavage de cette dernière par une solution aqueuse de chlorure de sodium.

Des résultats similaires sont obtenus quand la solution aqueuse de sel de fer mise en oeuvre conformément à l'invention contient 5 % de $FeCl_3$ en poids à la place du sel de Mohr.

En opérant comme il a été dit ci-dessus mais sous atmosphère d'air et non plus d'azote, il se forme du cyclooctanol à côté de la cyclooctanone dont la quantité formée reste inchangée. Ainsi, 24 heures après le mélange de $(E/H)_1$ avec $(E/H)_2$, 0,6 % du cyclooctane s'est encore une fois transformé en cyclooctanone tandis que 0,25 % du cyclooctane s'est transformé dans le même temps en cyclooctanol.

### Exemples 2 à 10 :

En opérant comme dans l'exemple 1 mais en remplaçant le cyclooctane par d'autres hydrocarbures, les résultats obtenus à 25°C, sous atmosphère d'azote, après une durée de 24 heures à partir du moment où $(E/H)_1$ est mélangée avec $(E/H)_2$, sont rassemblés dans le tableau unique ci-après.

## TABLEAU UNIQUE

| EXEMPLE N° | HYDROCARBURE | COMPOSES FORMES | SELECTIVITE | RENDEMENT EN PRODUITS D'OXYDATION PAR RAPPORT A H2O2 |
|---|---|---|---|---|
| 2 | Ethylbenzène | Acétophénone | 100 % | 8,5 % |
| 3 | Cumène | Phényl-2 propanol-2 | 100 % | 5 % |
| 4 | Tétraline | Tétralone<br>Tétralol | 50 %<br>50 % | 24 %<br>12 % |
| 5 | Cis-décaline | Cis-décalone-1 et<br>Cis-décalone-2<br>Cis et trans décalols-9 | 46 %<br>54 % | 4 %<br>2 % |
| 6 | Trans-décaline | Trans-décalone-1 et<br>Trans-décalone-2<br>Cis et trans décalols-9 | 74 %<br>26 % | 3-5 %<br>1 % |
| 7 | Cyclopentane | Cyclopentanone<br>Cyclopentanol | 45 %<br>55 % | 2 %<br>1 % |
| 8 | Cyclohexane | Cyclohexanone<br>Cyclohexanol | 47 %<br>53 % | 4 %<br>2-5 % |
| 9 | Cycloheptane | Cycloheptanone | 100 % | 3 % |
| 10 | Toluène | Benzaldéhyde | 100 % | 4 % |

EP 0 459 861 A1

Comme dans le cas du cyclooctane, la conversion des hydrocarbures ci-dessus en alcools et/ou cétones peut être poursuivie au-delà de celle indiquée dans le tableau ci-dessus sous l'effet d'additions supplémentaires de $H_2O_2$ dans le milieu de réaction.

Exemple 11 :

En opérant comme dans l'exemple 1 mais en remplaçant pour préparer $(E/H)_2$ la solution aqueuse à 5 % en poids de sel de Mohr par une solution de même concentration de $CuCl_2$, le cyclooctane après la durée de 24 heures est sélectivement transformé en cyclooctanone à raison de 0,2 %.

Exemple 12 :

En opérant comme dans l'exemple 1 mais en remplaçant la solution aqueuse à 5 % en poids de sel de Mohr par une hydrodispersion colloïdale contenant à l'état colloïdal 5 % en poids d'oxyde de fer $Fe_2O_3$ hydraté obtenue par vieillissement à 80°C d'une solution de chlorure ferrique acidifiée par l'acide chlorhydrique, le cyclooctane après la durée de 24 heures comme dans les autres exemples est sélectivement transformé en cyclooctanone à raison de 0,3 %.

Exemple 13 :

On opère comme à l'exemple 1 mais en modifiant comme suit la préparation de $(E/H)_2$ : à une solution de dioctylsulfosuccinate de sodium (2,4 g) dans 10 cm³ de cyclooctane, on ajoute goutte à goutte 0.5 cm³ d'une solution aqueuse à 5 % en poids de chlorure ferrique puis, après agitation 15 mn sous atmosphère inerte, toujours goutte à goutte 1 cm³ d'une solution aqueuse 0.1 N d'ammoniaque.

Exemple 14 :

On opère comme à l'exemple 1 mais en modifiant comme suit la préparation de $(E/H)_2$ : à une microémulsion obtenue par addition de 0.5 cm³ d'une solution aqueuse de nitrate ferrique à 5 % en poids dans une solution de dioctylsulfosuccinate de sodium (1.2 g) dans le cyclooctane (5 cm³), on ajoute sous atmosphère inerte une microémulsion préparée par addition lente de 1 cm³ d'une solution 0.1 N d'ammoniaque dans une solution de dioctylsufosuccinate de sodium (1.2 g) dans le cyclooctane (5 cm³).

**Revendications**

1. Procédé pour former un milieu dans lequel le peroxyde d'hydrogène agit sur un hydrocarbure dont au moins un atome de carbone porteur de liaisons C-H est à l'état d'hybridation de type $sp^3$, en présence d'un catalyseur, caractérisé en ce que l'on procède au mélange de deux microémulsions de type "eau dans huile" avec comme huile ledit hydrocarbure, la première contenant du peroxyde d'hydrogène, la seconde un sel ou un dérivé oxygéné colloïdal d'un métal de degré d'oxydation multiple et capable de conduire à des oxydes hydratés, de façon à obtenir une troisième microémulsion de même type que les deux premières.

2. Procédé selon la revendication 1, caractérisé en ce que le métal dont le sel ou un dérivé oxygéné colloïdal est présent dans la deuxième microémulsion est choisi parmi le fer, le cuivre, le ruthénium, le rhodium, le molybdène, le titane.

3. Procédé selon la revendication 2, caractérisé en ce qu'il est choisi comme sel de fer, le chlorure ferrique ou le sulfate double de fer et d'ammonium ou sel de Mohr et le nitrate ferrique.

4. Procédé selon la revendication 2, caractérisé en ce qu'il est choisi comme dérivé oxygéné colloïdal de fer celui obtenu sous forme de magnétite ou dispersion colloïdale aqueuse par vieillissement avec chauffage d'une solution aqueuse de chlorure ferrique suffisamment acidifiée par l'acide chlorhydrique pour que l'hydrolyse du chlorure ferrique soit initialement évitée à température ambiante ou ceux obtenus par hydrolyse des sels ferreux ou ferriques en mélange avec l'ammoniaque ou une autre base.

5. Procédé selon l'une des revendications 1 à 4, caractérisé en ce que l'hydrocarbure est liquide soit en l'état, soit par solubilisation dans un solvant organique, à une température comprise entre 10°C et la température d'ébullition du plus volatil des constituants de la troisième microémulsion.

6. Procédé selon la revendication 5, caractérisé en ce que l'hydrocarbure est liquide à une température comprise entre 10°C et 90°C.

7. Procédé selon la revendication 6, caractérisé en ce que l'hydrocarbure est choisi parmi le cyclooctane, le toluène, l'éthylbenzène, le cumène, la décaline, la tétraline, le cyclohexane, le cyclododécane.

8. Procédé selon l'une des revendications 5 et 6, caractérisé en ce que le solvant de l'hydrocarbure est le benzène ou des solvants chlorés.

9. Procédé selon l'une des revendications 1 à 8, caractérisé en ce que l'agent tensio-actif dans les microémulsions est ionique.

10. Procédé selon la revendication 9, caractérisé en ce que l'agent tensio-actif est le dioctylsulfosuccinate de sodium, l'acide benzènedodécylsufonique, le DTAB ou le DTAC.

11. Procédé selon l'une des revendications 1 à 10, caractérisé en ce que la première microémulsion est obtenue par mélange d'une solution de l'agent tensio-actif dans l'hydrocarbure avec une solution aqueuse de peroxyde d'hydrogène.

12. Procédé selon la revendication 11, caractérisé en ce que la solution de peroxyde d'hydrogène est ajoutée à la solution de l'agent tensio-actif dans l'hydrocarbure.

13. Procédé selon l'une des revendications 1 à 10, caractérisé en ce que la deuxième microémulsion est obtenue par mélange d'une solution de l'agent tensio-actif dans l'hydrocarbure avec une solution aqueuse d'un sel de métal ou avec une hydrodispersion d'un dérivé oxygéné colloïdal dudit métal en milieu aqueux ou en microémulsion.

14. Procédé selon la revendication 13, caractérisé en ce que soit la solution de sel soit la dispersion aqueuse de dérivé colloïdal soit la microémulsion contenant le dérivé colloïdal, sont mélangées à la solution de l'agent tensioactif dans l'hydrocarbure en étant ajoutées à elle.

15. Procédé selon l'une des revendications 11 à 14, caractérisé en ce que le mélange est réalisé à une température comprise entre 5°C et 90°C.

16. Procédé selon l'une des revendications 1 à 15, caractérisé en ce que :
    – la solution de l'agent tensio-actif dans l'hydrocarbure contient en poids une quantité dudit agent comprise entre 5 % et sa limite de solubilité dans l'hydrocarbure,
    – le métal est le fer,
    – la solution aqueuse de sel de fer comme la dispersion colloïdale de dérivé oxygéné colloïdal de fer, en milieu aqueux ou en microémulsion, contiennent en poids jusqu'à 10 % de sel de fer ou de dérivé oxygéné colloïdal de fer respectivement,
    – la solution de sel ou la dispersion colloïdale sont ajoutées à la solution de l'agent tensio-actif dans l'hydrocarbure, à raison, l'une ou l'autre par rapport à cette dernière, de 0,01 % à 20 % en poids,
    – la quantité d'eau dans la première microémulsion est telle que lors du mélange volume à volume de cette première microémulsion avec la deuxième pour former la troisième, elle représente jusqu'à deux fois la quantité d'eau apportée par la deuxième microémulsion.

17. Microémulsion telle qu'obtenue selon une des revendications 1 à 16.

18. Procédé d'activation oxydante de liaisons C-H dont l'atome de carbone est à l'état d'hybridation de type $sp^3$ dans un hydrocarbure par action de peroxyde d'hydrogène sur l'hydrocarbure en présence d'un catalyseur, caractérisé en ce que le peroxyde d'hydrogène agit sur l'hydrocarbure dans le milieu résultant, à l'état de microémulsion, du procédé selon l'une des revendications 1 à 16.

19. Procédé selon la revendication 18, caractérisé en ce que l'activation oxydante est poursuivie au-delà de celle assurée par le peroxyde d'hydrogène présent dans le milieu résultant du procédé selon l'une des revendications 1 à 16, par addition de peroxyde d'hydrogène au dit milieu alors encore ou pas à l'état de microémulsion.

20. Procédé selon l'une des revendications 18 et 19, caractérisé en ce que la température du milieu dans lequel le peroxyde d'hydrogène agit sur l'hydrocarbure est comprise entre 10°C et la température d'ébullition du plus volatil des constituants dudit milieu.

21. Procédé selon la revendication 19, caractérisé en ce que la température est comprise entre 10°C et 90°C.

**Office européen des brevets**

## RAPPORT DE RECHERCHE EUROPEENNE

Numero de la demande

EP   91 40 1293

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 288 337  (UNIVERSITE DU DROIT ET DE LA SANTE (LILLE II))<br>* Revendications; exemples 1-9 *<br>--- | 1-21 | C 07 B  41/06<br>C 07 B  41/02<br>C 07 C  49/385<br>C 07 C  49/78<br>C 07 C  47/54<br>C 07 C  35/00<br>C 07 C  33/22<br>C 07 C  45/28<br>C 07 C  29/48 |
| P,X | JOURNAL OF THE CHEMICAL SOCIETY, CHEMICAL COMMUNICATIONS, no. 17, 1 septembre 1990, pages 1193-1194; T. BRIFFAUD et al.: "Catalytic alkane activations in reverse microemulsions containing iron salts and hydrogen peroxide"<br>* En entier *<br>----- | 1-22 | |

**DOMAINES TECHNIQUES RECHERCHES (Int. Cl.5)**

C 07 B  41/00
C 07 C  27/00
C 07 C  29/00
C 07 C  45/00
C 07 C  47/00
C 07 C  49/00

Le présent rapport a été établi pour toutes les revendications

| Lieu de la recherche | Date d'achèvement de la recherche | Examinateur |
|---|---|---|
| LA HAYE | 27-08-1991 | WRIGHT M.W. |

CATEGORIE DES DOCUMENTS CITES

X : particullèrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

                 

& : membre de la même famille, document correspondant

EPO FORM 1503 03.82 (P0402)